# EUROPÄISCHE PATENTSCHRIFT

(11) **EP 0 757 978 B1**
(45) Veröffentlichungstag und Bekanntmachung des Hinweises auf die Patenterteilung: **12.09.2001**
(21) Anmeldenummer: 96111798.3
(22) Anmeldetag: 23.07.1996
(51) Int. Cl.: C07C 45/51, C07C 45/75, C07C 47/277

(54) **Verfahren zur Herstellung von 2-(Alkoxymethyl)acrolein**
Process for the preparation of 2-(alkoxymethyl)acrolein
Procédé pour la préparation de 2-(alkoxyméthyl)acroléine

(30) Priorität: 08.08.1995 DE 19529125
(43) Veröffentlichungstag der Anmeldung: 12.02.1997
(73) Patentinhaber: Degussa AG, 40474 Düsseldorf (DE)
(72) Erfinder: Höpp, Mathias, Dr., 63599 Biebergemünd (DE); Köhler, Klaus, 63512 Hainburg (DE); Arntz, Dietrich, Dr., 61440 Oberursel (DE)

(56) Entgegenhaltungen:
- US-A- 5 281 713

## Beschreibung

Die Erfindung betrifft ein Verfahren zur Herstellung von 2-(Alkoxymethyl)acrolein. Das Verfahren basiert auf der Umsetzung von Acrolein mit dem entsprechenden Alkohol und Formaldehyd in Gegenwart eines Katalysators.

Ein Verfahren zur Herstellung von 2-(Alkoxymethyl)-acrolein wird in den US-Patenten 5,281,713 und 5,177,266 beschrieben. Bei den in diesen Dokumenten beschriebenen Verfahren handelt es sich um eine zweistufige Reaktionsführung: In einer ersten Stufe wird ein C₁- bis C₆-Alkohol mit einer mindestens äquimolaren Menge Acrolein in Gegenwart einer Mineralsäure und einer katalytischen Menge eines trisubstituierten Amins in einem Lösungsmittel umgesetzt; bei dem Lösungsmittel handelt es sich um Wasser oder ein Gemisch aus Wasser und einem wasserlöslichen organischen Lösungsmittel. Das Reaktionsprodukt dieser ersten Stufe, 3-Alkoxypropionaldehyd, wird in der zweiten Stufe in Gegenwart einer Mineralsäure und einer katalytischen Menge eines disubstituierten Amins mit Formaldehyd in einem wäßrigen Reaktionsmilieu umgesetzt. Das gebildete 2-(Alkoxymethyl)acrolein wird mittels Extraktion aus der wäßrigen Phase abgetrennt, der Extrakt destillativ aufgearbeitet.

Das zuvor gewürdigte Verfahren weist eine Reihe von Nachteilen auf: Die Ausbeute ist niedrig; bei dem für die Weiterverarbeitung zu Herbiziden besonders wichtigen 2-(Methoxymethyl)acrolein wird die Ausbeute mit 44 % angegeben. Zudem ist die Raum-Zeit-Ausbeute des Verfahrens gering, da die Bildung der Zwischenstufe bereits eine Reaktionszeit von mehreren Stunden erfordert und zudem die extraktive Aufarbeitung des Reaktionsgemischs entsprechende Extraktionseinrichtungen erforderlich macht. Schließlich ist für die Extraktion zusätzlich ein organisches Lösungsmittel erforderlich, das sich in Wasser im wesentlichen nicht löst.

Die Aufgabe der vorliegenden Erfindung besteht demgemäß darin, das vorbekannte Verfahren bezüglich der Ausbeute und vorzugsweise auch der Raum-Zeit-Ausbeute zu verbessern. Eine weitere Aufgabe richtet sich darauf, den gesamten apparativen Aufwand zu vermindern und das Erfordernis eines Lösungsmittels für die Extraktion zu vermeiden.

Gefunden wurde ein Verfahren zur Herstellung von 2-(Alkoxymethyl)acrolein der allgemeinen Formel I 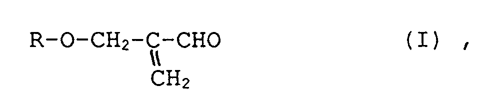 worin R eine Alkylgruppe mit 1 bis 6 C-Atomen ein cyclisches Alkyl oder eine Alkoxyalkylgruppe mit insgesamt 3 bis 6 C-Atomen bedeutet, aus Acrolein, einem primären oder Sekundören Alkohol der Formel ROH, worin R die vorgenannte Bedeutung hat, und einer Quelle für Formaldehyd, das dadurch gekennzeichnet ist, daß man Acrolein in Gegenwart einer mindestens äquimolaren Menge des Alkohols ROH und einer katalytischen Menge eines Katalysatorsystems auf der Basis eines sekundären Amins und einer Mineralsäure mit einer im wesentlichen äquimolaren Menge einer Quelle für Formaldehyd bei einem pH-Wert des Reaktionsgemischs im Bereich von 1 bis kleiner 7 in einer Stüfe umsetzt, wobei der Formaldehyd gegenüber Acrolein in geringem Unterschuß, etwa bis 10 Mol.-%, zweckmässigerweise aber in geringern Überschuß, bis zu 30 Mol.-% anwesend sein kann und das sekundäre Amin ein Amin der allgeneinen Formel R¹R²NH ist, wobei R¹R² Zweckmässigerweise lineares oder verzweigtes Alkyl mit 1 bis 6 C-Atomen, aber nicht tertiäres Alkyl sind. Das erfindungsgemäße Verfahren ist einstufig. Die Unteransprüche richten sich auf bevorzugte Ausführungsformen des Verfahrens.

Die Gruppe R in dem 2-(Alkoxymethyl)acrolein steht für eine Alkylgruppe mit 1 bis 6 C-Atomen, wobei die Alkylgruppe linear oder verzweigt sein kann. Das dem O-Atom des zu verwendenden Alkohols der Formel ROH benachbarte C-Atom ist primär oder sekundär. Besonders bevorzugt steht R für Methyl, Ethyl, n-Propyl, Iso-propyl, n-Butyl und Iso-butyl. R kann auch für ein cyclisches Alkyl, wie Cyclopentyl und Cyclohexyl, stehen. Sofern R eine Alkoxyalkylgruppe mit insgesamt 3 bis 6 C-Atomen ist, enthält die Alkylengruppe 2 bis 5 C-Atome und die Alkoxygruppe 1 bis 3 C-Atome.

Es wurde überraschenderweise gefunden, daß die separate Herstellung des 3-Alkoxypropionaldehyds nicht notwendig ist. Es reicht aus, die Zwischenstufe in situ zu erzeugen und sofort mit Formaldehyd weiter umzusetzen. Für beide Reaktionen, also die Alkoholaddition an Acrolein und die Mannich-Reaktion, welche im erfindungsgemäßen Verfahren nebeneinander ablaufen, wird dasselbe Katalysatorsystem, nämlich eine Kombination aus einem sekundären Amins und einer Mineralsäure, benutzt.

Bei dem sekundären Amin des Katalysatorsystems handelt es sich um ein Amin der allgemeinen Formel R¹R²NH, wobei R¹ und R² zweckmäßigerweise lineares oder verzweigtes Alkyl mit 1 bis 6 C-Atomen, vorzugsweise 2 bis 4 und insbesondere 4 C-Atomen, nicht aber tert.-Alkyl, sind. R¹ und R² können gemeinsam eine Alkylengruppe mit 4 oder 5 C-Atomen darstellen. Als Mineralsäure des Katalysatorsystems kommen insbesondere Schwefelsäure, Phosphorsäure, Salzsäure und Salpetersäure infrage, besonders bevorzugt wird jedoch Schwefelsäure. Die Einsatzmenge an sekundärem Amin und Mineralsäure liegt üblicherweise im Bereich zwischen 5 und 50 mMol, vorzugsweise zwischen 20 und 30 mMol, pro Mol-Acrolein. Das Mengenverhältnis zwischen dem sekundären Amin und der Mineralsäure wird derart gewählt, daß im Reaktionsgemisch aus dem Alkohol der Quelle für Formaldehyd und Acrolein ein pH-Wert im Bereich von 1 bis kleiner 7 resultiert. Vorzugsweise liegt der pH-Wert zwischen 3 und 6,5. Bezogen auf Acrolein wird der Alkohol in mindestens äquimolarem Verhältnis eingesetzt; bevorzugt wird jedoch ein Molverhältnis von Acrolein zu Alkohol von 1 zu 2 bis 1 zu 10. Besonders bevorzugt wird ein Molverhältnis von Acrolein zu Alkohol im Bereich von 1 zu 4 bis 1 zu 8.

Als Quelle für Formaldehyd eignen sich eine wäßrige oder alkoholische Formaldehydlösung, bevorzugt wird aber oligomerer oder polymerer Formaldehyd, wie er allgemein im Handel üblich ist; besonders bevorzugt wird Paraformaldehyd. Als weitere Quelle für Formaldehyd kommen unter den Reaktionsbedingungen depolymerisierbare Acetale von Formaldehyd infrage, wobei zweckmäßigerweise die Alkoholkomponente des Acetals identisch ist mit dem Alkohol, der Bestandteil des herzustellenden 2-(Alkoxymethyl)acroleins ist.

Acrolein und die Quelle für Formaldehyd werden im wesentlichen in äquimolarer Menge eingesetzt. Unter dem Begriff "im wesentlichen" wird hierbei verstanden, daß der aus der Quelle für Formaldehyd resultierende Formaldehyd gegenüber Acrolein in geringem Unterschuß, etwa bis 10 Mol-%, zweckmäßigerweise aber in geringem Überschuß, bis zu 30 Mol-%, vorzugsweise 5 bis 15 Mol-%, anwesend sein kann.

Das erfindungsgemäße Verfahren wird üblicherweise bei Temperaturen im Bereich zwischen 0 und 120 °C durchgeführt, bevorzugt wird jedoch eine Temperatur im Bereich zwischen 20 und 100 °C und besonders bevorzugt im Bereich zwischen 40 und 100 °C.

Gemäß einer bevorzugten Ausführungsform des erfindungsgemäßen Verfahrens wird zu einem Gemisch aus dem umzusetzenden Alkohol und dem Katalysatorsystem zunächst die Quelle für Formaldehyd, vorzugsweise eine wasserfreie Quelle für Formaldehyd und besonders bevorzugt Paraformaldehyd, zugegeben. Dieses Gemisch, das vorzugsweise einen pH-Wert von 3 bis 6,5 aufweist, wird auf die gewünschte Reaktionstemperatur, vorzugweise 40 bis 100 °C, gebracht. Anschließend wird Acrolein derart zudosiert, daß das Reaktionsgemisch niemals eine größere Konzentration an 3-Alkoxypropionaldehyd enthält, sondern dieser sofort zu dem gewünschten 2-(Alkoxymethyl)acrolein abreagiert. Der Gehalt an 3-Alkoxypropionaldehyd im Reaktionsgemisch liegt im allgemeinen deutlich unter 20 Mol-%, meistens deutlich unter 10 Mol-%, bezogen auf das zudosierte Acrolein. Unter den genannten Bedingungen ist die Reaktion auch mit Beendigung der Acrolein-Zudosierung bereits beendet.

Zur Aufarbeitung kann das Reaktionsgemisch direkt destilliert werden. Es ist auch möglich, das Reaktionsgemisch zunächst durch eine schnelle Destillation über einen Dünnschichtverdampfer von dem Katalysator abzutrennen. Durch eine anschließende Rektifikation wird überschüssiger Alkohol zurückgewonnen und dann das gewünschte 2-(Alkoxymethyl)acrolein isoliert. Im Falle des 2-(Methoxymethyl)acroleins wird dieses als Azeotrop mit anwesendem Wasser isoliert. Dieses Gemisch kann direkt für weitere Umsetzungen verwendet werden, bei denen Wasser nicht stört.

Zweckmäßigerweise wird die Destillationsvorlage mit geringen Mengen Säure, im allgemeinen 0,1 bis 1,0 Gew.-% Phosphor- oder Citronensäure, stabilisiert, um eine Polymerisation des Produkts durch mitdestillierte Spuren Amin auszuschließen.

Sofern erwünscht, kann das Azeotrop mittels üblicher Techniken, etwa azeotroper Entwässerung oder durch Pervaporation, entwässert und so das reine 2-(Alkoxymethyl)acrolein erhalten werden.

Wesentliche Vorteile des Verfahrens sind:
- die Einstufigkeit der Reaktionsführung
- verminderter apparativer Aufwand
- Entbehrlichkeit eines organischen Lösungsmittels durch Wegfall der Extraktionsstufe
- höhere Raum-Zeit-Ausbeute im Vergleich zu vorbekannten Verfahren
- höhere Ausbeute an 2-(Alkoxymethyl)acrolein bei der bevorzugten Ausführungsform.

### Beispiel 1: Herstellung von 2-(Methoxymethyl)acrolein

In einem Gemisch, bestehend aus Acrolein (58 g, 1 Mol), Methanol (224 g, 7 Mol), Dibutylamin (6,8 g, 0,029 Mol) und Schwefelsäure (2,5 g, 0,024 Mol) wird Paraformaldehyd (33 g, 1,1 Mol) suspendiert. Die Suspension wird für 2,5 h am Rückfluß erhitzt, bis der Paraformaldehyd weitgehend in Lösung gegangen ist und dabei abreagiert hat.

Anschließend wird direkt destilliert. Zunächst wird bei Normaldruck das überschüssige Methanol abdestilliert, dann bei einem Druck von 100 mbar und einer Temperatur von 34 bis 46 °C das Produkt im Gemisch mit Wasser und Methanol erhalten. Das Produkt wird mit 0,5 g Phosphorsäure stabilisiert. Der Gehalt wird per GC-Analyse bestimmt. Man erhält 90 g Produkt mit einem Gehalt von 50 % 2-(Methoxymethyl)acrolein; Ausbeute = 45 %.

### Beispiel 2: Herstellung von 2-(Methoxymethyl)acrolein

In einer Lösung von Dibutylamin (6,8 g, 0,029 Mol) und Schwefelsäure (2,5 g, 0,024 Mol) in Methanol (224 g, 7 Mol) wird Paraformaldehyd (33 g, 1,1 Mol) suspendiert. Die Suspension wird am Rückfluß erhitzt; über einen Zeitraum von 2 h wird kontinuierlich Acrolein (58 g, 1 Mol) zugetropft. Nach beendeter Zugabe wird noch 5 Minuten am Rückfluß erhitzt.

Anschließend wird direkt destilliert. Zunächst wird bei Normaldruck das überschüssige Methanol abdestilliert, dann bei einem Druck von 100 mbar und einer Temperatur von 40 bis 62 °C das Produkt im Gemisch mit Wasser und wenig Methanol erhalten. Das Destillat wird mit wenig Phosphorsäure und Hydrochinon stabilisiert. Der Gehalt wird per GC-Analyse bestimmt. Man erhält 117 g Produkt mit einem Gehalt von 64 % 2-(Methoxymethyl)acrolein; Ausbeute = 75 %.

### Beispiel 3: Herstellung von 2-(Methoxymethyl)acrolein

Ein Gemisch, bestehend aus Acrolein (58 g, 1 Mol), Methanol (224 g, 7 Mol), Dibutylamin (6,8 g, 0,029 Mol), Schwefelsäure (2,5 g, 0,024 Mol) und 37 %ige wäßrige Formaldehydlösung (89 g, 1,1 Mol) wird für 2,5 h am Rückfluß erhitzt.

Anschließend wird direkt destilliert. Zunächst wird bei Normaldruck das überschüssige Methanol abdestilliert, dann bei einem Druck von 100 mbar und einer Temperatur von 34 bis 46 °C das Produkt im Gemisch mit Wasser und Methanol erhalten. Das Produkt wird mit 0,5 g Phosphorsäure stabilisiert. Der Gehalt wird per GC-Analyse bestimmt. Man erhält 97 g Produkt mit einem Gehalt von 60 % Methoxymethylacrolein. Ausbeute = 58 %.

### Beispiel 4: Herstellung von 2-(Ethoxymethyl)acrolein

Die Umsetzung erfolgt analog zu Beispiel 2, wobei als Alkoholkomponente 7 Mol Ethanol eingesetzt werden. Nach destillativer Aufarbeitung erhält man 85 g 2-(Ethoxymethyl)acrolein, dies entspricht einer Ausbeute von 75 %.

### Beispiel 5: Herstellung von 2-(Isopropoxymethyl)acrolein

Die Umsetzung erfolgt analog zu Beispiel 2, wobei als Alkoholkomponente 7 Mol Isopropanol eingesetzt werden. Nach destillativer Aufarbeitung erhält man 87 g 2-(Isopropoxymethyl)acrolein, dies entspricht einer Ausbeute von 68 %.

## Patentansprüche

1. Verfahren zur Herstellung von 2-(Alkoxymethyl)acrolein der allgemeinen Formel I 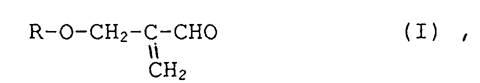 worin R eine Alkylgruppe mit 1 bis 6 C-Atomen ein cyclisches Alkyl oder eine Alkoxyalkylgruppe mit insgesamt 3 bis 6 C-Atomen bedeutet,
aus Acrolein, einem primären oder Sckeindären Alkohol der Formel ROH, worin R die vorgenannte Bedeutung hat, und einer Quelle für Formaldehyd,
**dadurch gekennzeichnet,**
**daß** man Acrolein in Gegenwart einer mindestens äquimolaren Menge des Alkohols ROH und einer katalytischen Menge eines Katalysatorsystems auf der Basis eines sekundären Amins und einer Mineralsäure mit einer im wesentlichen äquimolaren Menge einer Quelle für Formaldehyd bei einem pH-Wert des Reaktionsgemischs im Bereich von 1 bis kleiner 7 in einer Stüfe umsetzt, wobei der Formaldehyd gegenüber Acrolein in geringem Unterschuß, etwe bis 10 Mol.-%, zweckmässigerweise aber in geringem Überschuß, bis zu 30 Mol.-% anwesend sein kann und das sekundäre Amin ein Amin der allgemeinen Formel R¹R²NH ist, wobei R¹ und R² zweckmässigerweise lineares oder verzweigtes Alkyl mit 1 bis 6 C-atomen, aber nicht tertiäres Alkyl sind.

2. Verfahren nach Anspruch 1,
**dadurch gekennzeichnet,**
**daß** man als Alkohol einen primären oder sekundären Alkohol mit 1 bis 4 C-Atomen einsetzt.

3. Verfahren nach Anspruch 1 oder 2,
**dadurch gekennzeichnet,**
**daß** man als Quelle für Formaldehyd polymeren Formaldehyd einsetzt.

4. Verfahren nach einem der Ansprüche 1 bis 3,
**dadurch gekennzeichnet,**
**daß** man die Umsetzung bei einer Temperatur im Bereich zwischen 20 und 100 °C durchführt.

5. Verfahren nach einem der Ansprüche 1 bis 4,
**dadurch gekennzeichnet, daß** man die Umsetzung bei einem pH-Wert des Reaktionsgemischs im Bereich von 3 bis 6,5 durchführt.

6. Verfahren nach einem der Ansprüche 1 bis 5,
**dadurch gekennzeichnet, daß** man Acrolein und Alkohol der Formel ROH im Molverhältnis von 1 zu 2 bis 1 zu 10 einsetzt.

7. Verfahren nach einem der Ansprüche 1 bis 6,
**dadurch gekennzeichnet, daß** man zu einem Gemisch aus dem Alkohol und dem Katalysatorsystem zunächst die Quelle für Formaldehyd zugibt und anschließend Acrolein derart zudosiert, daß das Reaktionsgemisch stets weniger als 20 Mol-% 3-Alkoxypropionaldehyd, bezogen auf das zudosierte Acrolein, enthält.

8. Verfahren nach Anspruch 7,
**dadurch gekennzeichnet, daß** man Acrolein bei einer Temperatur im Bereich von 40 bis 100 °C bei einem pH-Wert von 3 bis 6,5 zudosiert.

9. Verfahren nach einem der Ansprüche 1 bis 8,
**dadurch gekennzeichnet,**
**daß** man das Reaktionsgemisch nach beendeter Umsetzung destillativ aufarbeitet.

## Claims

1. Process for the preparation of 2-(alkoxymethyl)acrolein corresponding to the general formula I 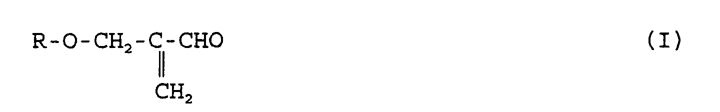 in which R denotes an alkyl group having 1 to 6 C atoms, a cyclic alkyl or an alkoxyalkyl group having a total of 3 to 6 C atoms,
from acrolein, a primary or secondary alcohol corresponding to the formula ROH in which R denotes the same as above, and a formaldehyde source,
**characterised in that**
in the presence of an at least equimolar quantity of the alcohol ROH and a catalytic quantity of a catalyst system based on a secondary amine and a mineral acid acrolein is reacted in one stage with a substantially equimolar quantity of a formaldehyde source at a pH of the reaction mixture within the range 1 to less than 7, wherein the formaldehyde may, vis-à-vis acrolein, be present in a slight deficiency, approximately up to 10 mol.%, however expediently in a slight excess, up to 30 mol.%, and the secondary amine is an amine corresponding to the general formula R¹R²NH wherein R¹ and R² are expediently linear or branched alkyl having 1 to 6 C atoms, however not tertiary alkyl.

2. Process according to Claim 1,
**characterised in that**
a primary or secondary alcohol having 1 to 4 C atoms is used as the alcohol.

3. Process according to Claim 1 or 2,
**characterised in that**
polymeric formaldehyde is used as the formaldehyde source.

4. Process according to one of Claims 1 to 3,
**characterised in that**
the reaction is carried out at a temperature within the range between 20 and 100°C.

5. Process according to one of Claims 1 to 4,
**characterised in that**
the reaction is carried out at a pH of the reaction mixture within the range 3 to 6.5.

6. Process according to one of Claims 1 to 5,
**characterised in that**
acrolein and alcohol corresponding to the formula ROH are used in a molar ratio of from 1 : 2 to 1 : 10.

7. Process according to one of Claims 1 to 6,
**characterised in that**
to a mixture of the alcohol and the catalyst system there is first admixed the formaldehyde source, followed by the dispensing of acrolein, such that the reaction mixture contains at all times less than 20 mol.% 3-alkoxypropionaldehyde, in relation to the acrolein dispensed.

8. Process according to Claim 7,
**characterised in that**
acrolein is dispensed at a temperature within the range 40 to 100°C at a pH of from 3 to 6.5.

9. Process according to one of Claims 1 to 8,
**characterised in that**
after the reaction has terminated the reaction mixture is worked up by distillation.

## Revendications

1. Procédé pour la préparation d'une 2-(alcoxyméthyl)acroléine de formule générale I 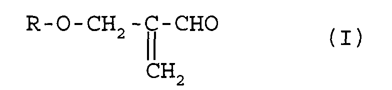 dans laquelle R représente un groupe alkyle ayant de 1 à 6 atomes de carbone, un groupe alkyle cyclique ou un groupe alcoxyalkyle ayant au total de 3 à 6 atomes de carbone,
à partir d'acroléine, d'un alcool primaire ou secondaire de formule ROH, dans laquelle R a la signification donnée précédemment, et d'une source de formaldéhyde,
**caractérisé en ce qu'**
on fait réagir en une étape l'acroléine avec une quantité pratiquement équimolaire d'une source de formaldéhyde, en présence d'une quantité au moins équimolaire de l'alcool ROH et d'une quantité catalytique d'un système catalytique à base d'une amine secondaire et d'un acide minéral, à un pH du mélange réactionnel dans la plage de 1 à < 7, le formaldéhyde pouvant être présent en faible défaut, d'environ jusqu'à 10 % en moles, mais convenablement en faible excès allant jusqu'à 30 % en moles, par rapport à l'acroléine, et l'amine secondaire étant une amine de formule générale R¹R²NH, R¹ et R² représentant convenablement un groupe alkyle linéaire ou ramifié ayant de 1 à 6 atomes de carbone, mais non un groupe alkyle tertiaire.

2. Procédé selon la revendication 1,
**caractérisé en ce qu'**
on utilise comme alcool un alcool primaire ou secondaire ayant de 1 à 4 atomes de carbone.

3. Procédé selon la revendication 1 ou 2,
**caractérisé en ce qu'**
on utilise comme source de formaldéhyde un formaldéhyde polymère.

4. Procédé selon l'une quelconque des revendications 1 à 3,
**caractérisé en ce que**
la réaction est effectuée à une température dans la plage comprise entre 20 et 100°C.

5. Procédé selon l'une quelconque des revendications 1 à 4,
**caractérisé en ce qu'**
on effectue la réaction à un pH du mélange réactionnel dans la plage de 3 à 6,5.

6. Procédé selon l'une quelconque des revendications 1 à 5,
**caractérisé en ce qu'**
on utilise l'acroléine et l'alcool de formule ROH en un rapport molaire allant de 1:2 à 1:10.

7. Procédé selon l'une quelconque des revendications 1 à 6,
**caractérisé en ce qu'**
on ajoute d'abord la source de formaldéhyde à un mélange de l'alcool et du système catalytique, et on y ajoute ensuite de façon réglée l'acroléine, de manière que le mélange réactionnel contienne toujours moins de 20 % en moles de 3-alcoxypropionaldéhyde, par rapport à l'acroléine ajoutée.

8. Procédé selon la revendication 7,
**caractérisé en ce qu'**
on ajoute de façon réglée l'acroléine à une température dans la plage de 40 à 100°C, et à un pH de 3 à 6,5.

9. Procédé selon l'une quelconque des revendications 1 à 8,
**caractérisé en ce qu'**
une fois la réaction terminée, le mélange réactionnel est soumis à un traitement final par distillation.
